# EUROPEAN PATENT SPECIFICATION

(11) **EP 1 146 891 B1**
(45) Date of publication and mention of the grant of the patent: **20.12.2006**
(21) Application number: 00903106.3
(22) Date of filing: 05.01.2000
(51) Int. Cl.: A61K 38/17

(54) **THERAPY OF RESTENOSIS AND ATHEROSCLEROSIS WITH A SOLUBLE VEGF RECEPTOR AND ANGIOPOEITIN-1**
BEHANDLUNG VON RESTENOSE UND ATHEROSKLEROSE MIT EINEM LÖSLICHEN VEGF REZEPTOR UND ANGIOPOIETIN-1
THERAPIE DE LA RESTENOSE ET DE L' ARTERIOSCLEROSE AVEC UN RECEPTEUR AU VEGF SOLUBLE ET L' ANGIOPOIETINE-1

(30) Priority: 15.01.1999 US 115977 P
(43) Date of publication of application: 24.10.2001
(73) Proprietor: Medstar Research Institute, Washington, DC 20010 (US)
(72) Inventor: EPSTEIN, Stephen, E., Rockville, MD 20852 (US); KORNOWSKI, Ran, Rockville, MD 20852 (US); FUCHS, Shmuel, Rockville, MD 20852 (US); LEON, Martin, Bethesda, MD 20817 (US)
(74) Representative: Plougmann & Vingtoft A/S
(86) International application number: PCT/US2000/000161
(87) International publication number: WO 2000/041712

(56) References cited:
- WO-A-98/33510
- KOBLIZEK T I ET AL: "ANGIOPOIETIN-1 INDUCES SPROUTING ANGIOGENESIS IN VITRO" CURRENT BIOLOGY, CURRENT SCIENCE,, GB, vol. 8, no. 9, 1998, pages 529-532, XP000972727 ISSN: 0960-9822
- KORPELAINEN EIJA I ET AL: "Signaling angiogenesis and lymphangiogenesis." CURRENT OPINION IN CELL BIOLOGY, vol. 10, no. 2, April 1998 (1998-04), pages 159-164, XP002197940 ISSN: 0955-0674
- LAZAROUS ET AL.: 'Comparative Effects of Basic Fibrblast Growth factor and Vascular Endothelial Growth factor on Coronary Collateral Development and the Artierial Response to Injury' CIRCULATION, vol. 94, no. 5, 01 September 1996, pages 1074 - 1082, XP002927902
- NAMBU ET AL: 'Angiopoietin 1 inhibits ocular neovascularization and breakdown of the blood-retinal barrier' GENE THERAPY vol. 11, 2004, pages 865 - 873

## Description

### FIELD OF THE INVENTION

The present invention relates to compositions for treatment of restenosis and/or atherosclerosis.

The present invention further relates to compositions for inhibiting the development of miaovessels within the wall of coronary and/or peripheral vessels.

Microvessels can develop in response to angioplasty procedures and/or stent implantation, and develop during the development of atherosclerosis; and thus, the present invention relates to compositions and methods for inhibiting the development of microvessels within the wall ofcoronary and/or peripheral vessels in response to angioplasty procedures and/or stent implantation, and during the development of atherosclerosis

The present invention further relates to compositions for inhibiting the development of microvessels within the wall of coronary and/or peripheral vessels in response to angioplasty procedures and/or stent implantation, and during the development of atherosclerosis, for treating restenosis and/or atherosclerosis.

Accordingly, the present invention relates to compositions for administering the soluble VEGF receptor, which inhibits the family of FGFs and their signalling cascades, and ang 1 which induces vessel maturation. The administration can be sequential, simultaneous, or separated by a desired time period and can be by any suitable means.

Accordingly, the present invention relates to protein delivery to treat restenosis and/or atherosclerosis. The present invention relates to such protein delivery to inhibit the development of microvessels (vasovasorum). The present invention relates to such protein delivery for anti-angiogenesis, e.g., to suppress angiogenesis; for instance, to thereby inhibit or prevent or prolong the onset of restenosis and/or atherosclerosis.

In particular, the invention pertains to a composition and a kit from treating restenosis and/or atherosclerosis comprising the soluble VEGF receptor and angiopoietin-1. Additionally, the invention provides the use of the soluble VEGF receptor and angiopoietin-1 for the preparation of a composition for treating restenosis and/or atherosclerosis.

None of the documents cited in the following text is admitted to be prior art with respect to the present invention.

### BACKGROUND OF THE INVENTION

As discussed generally by Jean Marx at page 320 of Science, Vol. 265 (July 15, 1994), each year about 330,000 patients in the United States undergo coronary and/or peripheral angioplasty, a procedure designed to open up blood vessels, e.g., coronary arteries, clogged by dangerous atherosclerotic plaques (atherosclerosis) and thereby restore normal blood flow. For a majority of these patients, the procedure works as intended. Nearly 33% of these patients (and maybe more by some accounts), however, develop restenosis, wherein the treated arteries become quickly clogged again. These patients are no better off, and sometimes worse off, than they were before angioplasty. Excessive proliferation of smooth muscle cells in blood vessel walls contributes to restenosis.

While the use of stents has appreciably reduced the rate of restenosis, even with this treatment, restenosis occurs in 5 to 20% of patients. Thus, the problem of restenosis is formidable, despite recent advances in reducing its incidence.

Two primary mechanisms appear to be involved in the development of restenosis.

First, recoil of the vessel wall (negative remodeling) leads to gradual narrowing of the vessel lumen..

Second, an exaggerated healing response of medial and/or adventitial smooth muscle cells (SMCs) to vascular injury, which involves the excessive proliferation of SMCs and the migration of SMCs to the subintima, where they continue to proliferate and begin to secrete extracellular matrix.

These processes involving SMCs cause the neointimal mass to expand and gradually encroach upon the coronary lumen; ultimately the expanding lesion narrows the vessel, increases resistance to blood flow, and causes ischemic symptoms.

In the absence of stenting, both remodeling and an expanding neointima contribute to restenosis; when stents are deployed negative vascular remodeling is prevented and restenosis occurs only as a result of the expanding neointimal mass.

Given these pathophysiologic mechanisms, the problem of controlling restenosis becomes largely the problem of controlling the development of the neointimal mass and, in the absence of stenting, also in controlling the amount of negative vascular remodeling.

The potential role of the vasovasorum as a determinant of atherosclerotic plaque mass was first raised by the studies of Barger et al., 1984 (Barger et al., "Hypothesis: vasavasorum and neovascularization of human coronary arteries. A possible role in the pathophysiology of atherosclerosis." N Eng J Med 310(3):175-7 (Jan. 1984)). These investigators demonstrated that atherosclerotic plaques were highly vascularized. In particular, the mass of vasovasorum microvessels present in the wall of the coronary artery at the site of atherosclerotic plaque was found to be increased roughly in proportion to plaque mass.

This interesting observation could not however, distinguish between two alternative possibilities: 1) that the angiogenic stimulus derived in some way from the growing plaque, versus 2) that increasing angiogenesis causes plaque growth. The latter possibility was suggested to play a role by the recent study from Folkman's laboratory.

Dr. Folkman and his colleagues demonstrated that in apoE knockout mice, which are prone to develop atherosclerosis, treatment with endostatin (a potent anti-angiogenic drug) reduces the magnitude of plaque mass development. This finding was accompanied by a decrease in the number of blood vessels supplying the plaque. Thus, these results are compatible with the concept that atherosclerotic plaque growth is limited by its blood supply, and therefore determined by angiogenesis processes involving the vasovasorum. Dr. Folkman published an abstract of the work in the November 1998 edition of Circulation, and presented his findings at the TCT Symposium in Washington in October 1998, and at the American Heart Association Annual Meeting in Dallas, in November 1998.

Angiogenesis involves the sprouting of capillaries from preexisting blood vessels and/or the development of new vessels. This process is controlled by the action of several angiogenic growth factors and their tyrosine kinase receptors. Currently, the basic mechanisms responsible for angiogenesis are not fully understood.

However, two systems involving vascular endothelial growth factor (VEGF) and the angiopoietin-1/angiopoietin-2 ligands, along with their specific receptors (VEGF-R1, VEGF-R2 and Tie-2 respectively), seem to have a unique and specific roll in the induction and maintenance of new blood vessel formation. Studies in mice carrying homozygous disruption in these receptors have demonstrated VEGF and angopoietin-1 act in sequence; 1) VEGF, through VEGF-R1, induces endothelial cell-cell interaction, proliferation, and tube formation; 2) angiopoictin-1, through binding to its receptor Tie-2, elicits recruitment and interaction with peri-endothelial support cells, thus maintaining vessel integrity and stabilizing newly formed blood vessels. Angiopoietin-2 appears to be a functional antagonist of antiopoictin-1, and its expression may be a necessary step in destabilizing an existing vessel, thereby allowing it to initiate new vascular buds and branches.

Accordingly, improvements in the therapy, prophylaxis and diagnosis of restenosis and/or atherosclerosis, especially in compositions therefor and methods thereof, would be an advance over the state of the art.

Reference is made to WO 98/33510, Kwon et al., Journal of Clinical Investigation, 101(8): 1551-56 (April 1998), Kwon et al., "Adventitial Vasa Vasorum in Balloon-injured Coronary Arteries: Visualization and Quantitation by a Microscopic Three-dimensional Computed Tomography Technique," J. Am. Coll. Cardiol. (1998), Inoue et al., Circulation 98:2108-2116 (November 1998), and Asahara et al., Circ Res 83(3):233-240 (August 1998).

WO 98/33510 relates to restenosis and/or atherosclerosis diagnosis, prevention and therapy, e.g., by decreasing viral load; and, the reader is respectfully directed to that document for information and literature citations involving restenosis and/or atherosclerosis diagnosis, prevention and therapy. The Kwon et al articles provide a visualization and quantitation of three-dimensional spacial patterns of vaso vasorum in normal and balloon injured or hypercholesterolemic porcine coronary arteries. Asahara et al. relates to the effects of angiopoietin on postnatal neovascularization. And, Inoue et al. is directed to VEGF expression in atherosclerotic lesions.

Further, mention is made of Takahashi et al. Jpn J Cancer Res 89(4):445-51 (April 1998) which may relate to clotrimazole, an imidazole antimycotic, as an inhibitor of angiogenesis and Raymond Presse Med 27(24):1221-4 (July 1998) which discusses antiangiogenic properties of endostatin and angiostatin, and cites Folkman, Nature 390:404-7 (1997). These documents do not appear to directly address inhibiting VEGF and inducing vessel maturation, for instance, for preventing or treating restenosis and/or atherosclerosis, in contrast to the present invention.

Accordingly, in general, as a contrast to the foregoing documents and those cited otherwise herein and that which is believed to have been the knowledge in the art, the present invention addresses restenosis and/or atherosclerosis prevention and/or therapy by inhibiting the specific ligands, receptors, and/or their signalling cascades that have been identified as the natural pathways by which new vessels develop. Thus, the present invention inhibits microvessel development by inhibiting VEGF or its activity and by inducing vessel maturation by administering ang-1. It also inhibits microvessel development by inhibiting ang-2, which is believed necessary to destabilize a mature vessel, thereby preparing it for new vessel budding and branching. This invention, in its totality, is seen as providing improvements in the therapy of restenosis and/or atherosclerosis, especially in providing compositions therefor; and thus, the present invention is seen as an advance over the stale of the art.

Implication of VEGF receptors and angiopoietin-1 in the formation of blood vessels has been investigated by Koblizek et al. Current Biology 1998, 8, 529-532. The authors show that ang 1 is a potent inducer of sprouting angiogenesis and suggest that inhibition of its activity may have implications for therapeutic intervention of pathological angiogenesis.

Korpelainen and Alitalo review signalling pathways involved in angiogenesis and lymphangiogenesis, including signalling through receptors of the VEGF and Tie families.

WO 98/33510 discloses a composition for therapy for restenosis and/or atherosclerosis comprising an agent for decreasing viral load of cytomegalovirus.

Thus, the combined use of the soluble VEGF receptor and angiopoietin-1 in the treatment of restenosis or atherosclerosis has not been suggested in the art.

### OBJECTS AND SUMMARY OF THE INVENTION

It is therefore an object of the invention to provide compositions for the therapy for restenosis and/or atherosclerosis.

It is yet a further object of the invention to provide such compositions for therapy which comprise the soluble VEGF receptor and ang 1.

It is a yet further object of the invention to provide such compositions in conjunction with additional compositions.

The present invention thus provides compositions for the therapy for restenosis and/or atherosclerosis.

The administration can be after angioplasty, coronary and/or peripheral angioplasty, to prevent the development of, or to provide treatment for, atherosclerosis and/or restenosis. The angioplasty procedure could involve any of the types of angioplasty (e.g. balloon, atherectomy, laser) employed either with or without a stent. Thus, the invention provides compositions for treatment of atherosclerosis and/or restenosis.

Similarly, the compositions of the invention can be administered before, during, or after any type of angioplasty procedure; before angioplasty, to prevent, i.e., as a prophylaxis against, restenosis and/or atherosclerosis. They can also be administered any time during the lifetime of the individual from childhood to adulthood, to prevent the development or progression of atherosclerosis.

Subcutaneous, intradermal or intramuscular administration are presently preferred. Direct administration to blood vessels (including via catheter-based systems and via by direct intra-arterial infusion) are also encompassed within the invention (see, e.g., Epstein et al., JACC VoL 23, No. 6, 1994:1278-88) Chang et al., Science 267:518-22 (January 27, 1995) and; WO9605321. The invention further comprehends methods for preparing the compositions of the invention, as well as kits for compositions and methods of the invention. For instance, the invention comprehends a kit comprising the soluble VEGF receptor and angiopoietin the agents can be in separate containers; the agents can be in separate containers contained in a package; and, the kit can optionally include instructions for the storage and/or use and/or administration of the agents.

The terms "comprises", "comprising", and the like can have the meaning ascribed to them in U.S. Patent Law and can mean "includes", "including" and the like.

These and other embodiments are disclosed or are obvious from and encompassed by, the following Detailed Description.

### BRIEF DESCRIPTION OF FIGURES

The following Detailed Description, given by way of example, but not intended to limit the invention to specific embodiments described, maybe understood in conjunction with the accompanying Figures, incorporated herein by reference, in which:
Figure 1 shows the microvascular angiogenic processes that occur during restenosis and atherosclerosis; and
Figure 2 shows the reduction of microangiogenesis by the compositions and methods of the invention.

(With respect to the Figures, reference is made to Suri et al., "Requisite Role of Angiopoietin-1, a Ligand for the TIE2 Receptor, during Embryonic Angiogenesis." Cell 87:1171-80 (Dec. 1996), as the present inventors, as part of the present invention, adapted the accompanying Figures therefrom.)

### DETAILED DESCRIPTION

This invention is designed to employ protein delivery to treat restenosis, by inhibiting the development of microvessels (vasovasorum) in the injured vessel, insofar as angiogenesis occurring within the vessel wall is an important permissive determinant of neointimal development, and or vessel remodeling. The invention uses various anti-angiogenesis strategies to suppress angiogenesis, and thereby inhibit the development of restenosis and/or atherosclerosis.

Angiogenesis involves the sprouting of capillaries from preexisting blood vessels and/or the development of new vessels. This process is controlled by the action of several angiogenic growth factors and their tyrosine kinase receptors. Two systems involving vascular endothelial growth factor (VEGF) and the angiopoietin-1 ligands, along with their specific receptors (VEGF-R1, VEGF-R2 and Tie-2 respectively), seem to have a unique and specific roll in the induction and maintenance of new blood vessel formation. The angiopoietin-2 ligand also appears to play an important role in the cascade of events leading to angiogenesis. Studies in mice carrying homozygous disruption in these receptors have demonstrated VEGF and angiopoietin-1 act in sequence: 1) VEGF, through VEGF-R1, induces endothelial cell-cell interaction, proliferation, and tube formation; 2) angiopoietin-1, through binding to its receptor Tie-2, elicits recruitment and interaction with peri-endothelial support cells, thus maintaining vessel integrity and stabilizing newly formed blood vessels. Angiopoietin-2 appears to be a functional antagonist of antiopoietin-1; ang-2 expression may be a necessary step in destabilizing an existing vessel, thereby allowing it to initiate new vascular buds and branches. Multiple studies have also demonstrated that members of the family of FGFs and their signaling cascades stimulate angiogenesis (see, e.g., Flugelman et al., Circulation 88(6):2493-500 (Dec. 1993)). bFGF and aFGF are in lesions, suggesting that they may play a role in expansion of vasovasorum. It therefore appears that each of these ligands, through their specific receptors, control a specific, complementary function relating to endothelial cells that collectively accounts for a significant part of endothelial cell morphogenesis into mature, functional blood vessels.

An analysis of the many cellular and molecular mechanisms involved in atherogenesis reveals a remarkable parallelism to the cellular and molecular mechanisms involved in restenosis. On these bases, it would appear that strategies designed to inhibit angiogenesis involving the vasovasorum in the patient undergoing angioplasty would, just as in atherosclerosis, also inhibit processes leading to restenosis. The end result would therefore be to inhibit restenosis.

A strategy employed by the present invention is based on the concept that a critical rate-limiting step in restenosis development is the vascular supply of the injured vessel; that neointimal growth, and possibly the amount of negative vascular remodeling, are dependent on the development of a greater number of the blood vessels constituting the vasovasorum, an angiogenic process that can be modulated by anti-angiogenic interventions.

Targets for anti-angiogenic strategies are VEGF, its receptors, and its signaling cascade, bFGF its receptors, and its signalling cascade; and angiopoeitin-1, its receptor, and its signaling cascade, angiopoeitin-2, its receptor, and its signaling cascade.

Delivery to patient will vary depending on the clinical situation as described in the following situations.

Before, during, or following angioplasty, the anti-angiogenic factor could be administered systemically, either orally or intravenously. It could also be administered directly into the coronary artery in patients undergoing coronary angioplasty, or into the artery supplying the leg in patients undergoing peripheral vessel angioplasty. The anti-angiogenic factor could be applied directly to the wall of the injured vessel via either. 1. a ballon catheter that allows administration of the anti-angiogenic factor directly into the media and/or adventitia, or 2. a stent that has been deployed and which releases the factor into the vessel wall.

Thus, the present invention includes compositions for treating restenosis and/or atherosclerosis. The present invention includes compositions comprising the soluble VEGF receptor.

The use of the soluble VEGF receptors and angiopoietin-1 for the preparation of a composition for treating restenosis and/or atherosclerosis.

Angioplasty represents an acute injury model and the present invention is based on findings that many of the processes leading to neointimal development following angioplasty are the same that lead to atherosclerotic plaque development. Studies of cadaver hearts revealed marked development of the vasovasorum of the walls of coronary arteries contiguous to atherosclerotic plaque. During angiogenesis in apoE knockout mice, a considerable number of plaque microvessels were observed in growing atheromata. Administration of endostatin to apoE knockout mice retarded the progression of plague growth, a change associated with a decrease in the amount of microvessels present in the plaque. The inventors have reviewed many specimens of balloon injured porcine coronary arteries and stented porcine coronary arteries and have found that there is a marked angiogenic response involving microvessels of both the adventitia and the neointimal at the site of the vessel injury.

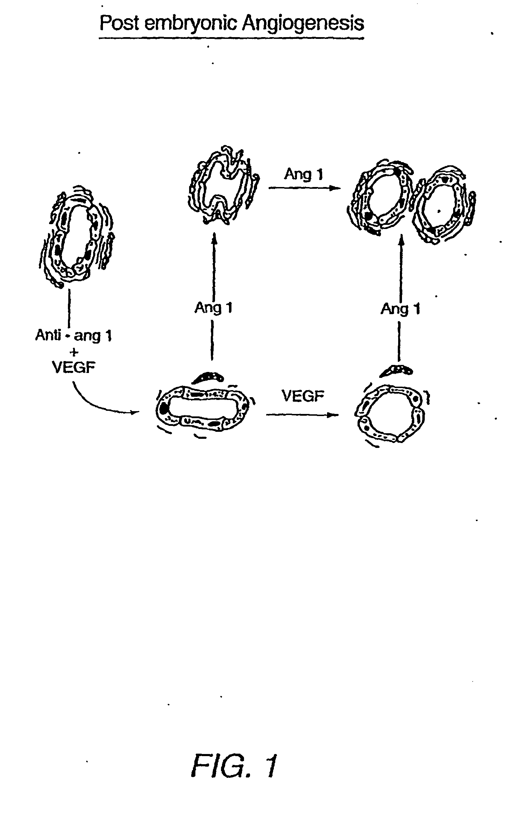

Accordingly, microvascular angiogenesis (expansion of the vasovasorum) occurs during both atherogenesis and during restenosis. The coordinated sequential expression of VEGF and ang-1, and perhaps ang-2, with activation of their signaling cascades, are consistent components of the post embryonic microvascular angiogenic processes that occur during restenosis and atherosclerosis (note for instance Figure 1: upregulation of VEGF is necessary to destabilize a mature vessel to enable it to begin the angiogenic process, and upregulation of angiopoietin 1 (ang-1) induces vessel maturation).

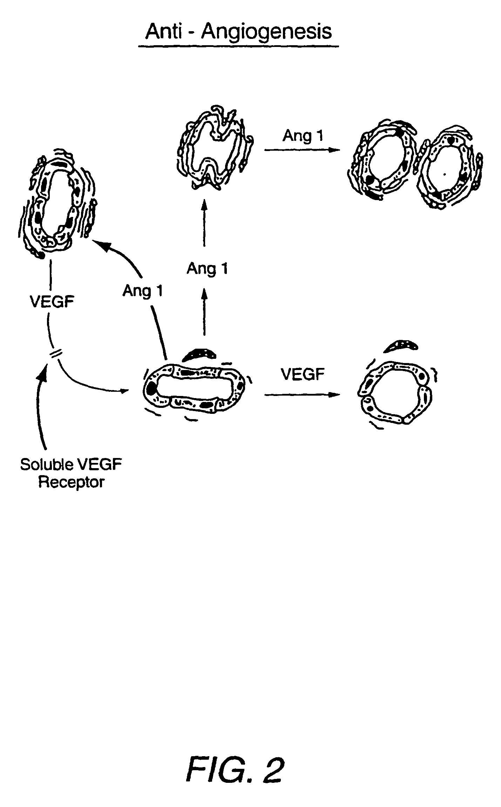

From this, administration of the soluble VEGF receptor and (ang-1 or an agent which induces ang-1) reduces microangiogenesis (Figure 2) and, thereby, inhibits atherosclerosis (e.g., as shown by the apoE knockout mouse model) and reduces restenosis (e.g., by the porcine coronary artery injury model).

With respect to ang-1 it is noted that VEGF and angiopoietins, along with their receptors are important regulators (Koblizek et al. Curr Biol 8(9):529-32 (April 1998)). Ang-1 and ang-2 modulate VEGF (Asahara et al. Cir Res 83(3):233-40 (August 1998)). Ang-2 has been recognized as an antagonist for ang-1 and Tie-2 (Maisonpierre et al. Science 277(5322):55-60 (July 1997)). Also, it has been observed that excess soluble Tie-2 abolishes the chemotactic response of endothelial cells towards ang-1; and that ang-2 dose-dependently blocks directed migration towards ang-1, consistent with ang-2 being an inhibitor of ing-1 (Witzenbichler et al. J Biol Chem 273(29):18514-21 (July 1998)).

Further, ang-1 has been cloned and plays a mediating role; for instance, mice engineered to lack ang-1 display angiogenic deficits (Suri et al. Cell 87(7): 1171-80 (December 1996)). Transgenic expression, e.g., overexpression of ang-1 in mice has been demonstrated (Suri et al., Science 282(5398):469-71 (October 1998)). And, ang-1 and ang-2 genes have been localized to human genes 8q223-q23 and 8p23 (Cheung et al. Genomics 48(3):389-91 (March 1998)).

Accordingly, using the knowledge of ang-1 having been cloned, that transgenic expression of ang-1 has been demonstrated, and that the ang-1 and ang-2 genes have been localized, to obtain administration of an agent which induces vessel maturation, such as ang-1 or an agent which induces ang-1, no undue experimentation is necessary, as the expression of ang-1 can be performed either *in vivo* or *in vitro*; and, one can otherwise obtain ang-1 for administration. Thus, the invention comprehends administration of ang-1.

As to VEGF, it is noted that VEGF induces angiogenesis by binding to VEGF-receptor-2 tyrosine kinase or VEGFR2 TK. The VEGFR2 TK catalytic domain has been cloned and expressed via a baculovirus expression system; Cd2+ was found to be an inhibitor of the enzyme, with inhibition competitive with respect to Mg2+ and non competitive with respect to MgATP (Parast et al. Biochemistry 37(47):16788-801 (November 1998); see also Pepper et al. J Cell Physiol 177(3):439-52 (December 1998) (by acting in concert bFGF or VEGF, VEGF-C has a potent synergistic effect on the induction of angiogenesis and VEGF, bFGF and VEGF-C are capable of altering endothelial cell extracellular proteolytic activity).

Also, it has been reported that ERK1/2 is necessary for VEGF-induced endothelial cell proliferation; and that MAPK kinase inhibitors abolished ERK1/2 activation in a concentration-dependent manner (Parenti et aL J Biol Chem 272(7):4220-6 (February 1998)). 8-(3-oxo-4,5,6-trihydroxy-3h-xanthen-9-yl)-1-naphthoic acid inhibited binding of VEGF to VEGFR-2 or VEGFR-1 as well as MAPK phosphorylation induced by VEGF and could be an inhibitor ofVEGF and basic FGF signal transduction (Igarashi et al. Int J Mol Med 2(2):211-215 (August 1998)).

Further, VEGF and its receptors (VEGFR-1 and VEGFR-2) as well as ang-1 and its receptor Tie-2 are key transduction systems involved in the regulation of embryonic vascular development; and, inhibition of the VEGF signal transduction resulted in inhibition of neovascularization in angiogenesis-dependent diseases such as proliferative retinopathy or solid tumor growth and the VEGF signal transduction system is useful as anti-angiogenic therapy (Breier et al. Thromb Haemost 78(1):678-83 (July 1997); see also Metais et al. Am J. Physiol 275(4 Pt 2):H1411-8 (October 1998) (effects of coronary artery disease on expression and microvascular response to VEGF)).

As to administration of the soluble VEGF receptor), and the angiopoietin-1 these agents can be administered by any suitable means, and such means can include the proteins, an angioplasty balloon, a catheter-type device that facilitates delivery of the agent(s) to the vessel wall, or intra-arterial infusion (See Witzenbichler et al. Am J Pathol 153(2):381-94 (August 1998): VEGF-C promotes angiogenesis; demonstrates administration of VEGF-C by means of naked plasmid DNA (pcVEGF-C 500 microg), polymer coating of an angioplasty balloon (n=8) or as a recombinant human protein (rhVEGF-C 500 microg) by direct infra-arterial infusion; WO 98/33510 (vectors including viral vectors, plasmid vectors)).

The soluble VEGF receptor and angiopoietin-1 can be obtained by purification from natural sources or from purification from recombinant sources; and, techniques for such purifications or for protein purification are generally known and require no undue experimentation by the skilled artisan.

The methods for making a vector or recombinant for expression of such agents either *in vivo* or *in vitro* can be by or analogous to the methods disclosed in: U.S. Patent Nos. 4,603,112, 4,769,330, 5,174,993, 5,505,941, 5,338,683, 5,494,807,4,722,848, WO 94/16716, WO 96/39491, Paoletti. "Applications of pox virus vectors to vaccination: An update," PNAS USA 93:11349-11353, October 1996, Moss, "Genetically engineered poxviruses for recombinant gene expression, vaccination, and safety," PNAS USA 93:11341-11348, October 1996, Smith et al., U.S. Patent No. 4,745,051 (recombinant baculovirus), Richardson, C.D. (Editor), Methods in Molecular Biology 39, "Baculovirus Expression Protocols" (1995 Humana Press Inc.); Smith et al., "Production of Huma Beta Interferon in Insect Cells Infected with a Baculovirus Expression Vector," Molecular and Cellular Biology, Dec., 1983, Vol. 3, No. 12, p. 2156-2165; Pennock et al., "Strong and Regulated Expression of *Escherichia coli* B-Galadosidase in Infect Cells with a Baculovirus vector," Molecular and Cellular Biology Mar. 1984. Vol. 4, No. 3, p. 399-406; EPA 0 370 573, U.S. application Serial No. 920,197, filed October 16,1986, EP Patent publication No. 265785, U.S. Patent No. 4,769,331 (recombinant herpesvirus), Roizman, "The function of herpes simplex virus genes: A primer for genetic engineering of novel vectors," PNAS USA 93:11307-11312, October 1996, Andreansky et al., "The application of genetically engineered herpes simplex viruses to the treatment of experimental brain tumors," PNAS USA 93:11313-11318, October 1996, Robertson et al. "Epstein-Barr virus vectors for gene delivery to B lymphocytes," PNAS USA 93:11334-11340, October 1996, Frolov et al., "Alphavirus-based expression vectors: Strategies and applications," PNAS USA 93:11371-11377, October 1996, Kitson et al., J. Virol. 65, 3068-3075, 1991; U.S. Patent Nos. 5,591,439, 5,552,143 (recombinant adenovirus), Grunhaus et al., 1992, "Adenovirus as cloning vectors," Seminars in Virology (Vol. 3) p. 237-52, 1993, Ballay et al. EMBO Journal, vol. 4, p. 3861-65, Graham, Tibtech 8, 85-87, April, 1990, Prevec et al., J. Gen Virol. 70, 429-434, PCT WO91/11525, Felgner et al. (1994), J. Biol. Chem. 269, 2550-2561, Science, 259:1745-49, 1993 and McClements et al., "Immunization with DNA vaccines encoding glycoprotein D or glycoprotein B, alone or in combination, induces protective immunity in animal models of herpes simplex virus-2 disease," PNAS USA 93:11414-11420, October 1996, and U.S. Patents Nos 5,591,639, 5,589,466, and 5,580,859 relating to DNA expression vectors, *inter alia.* See also WO 98/33510; Ju et al., Diabetologia, 41:736-739, 1998 (lentiviral expression system); Sanford et al., U.S. Patent No. 4,945,050 (method for transporting substances into living cells and tissues and apparatus therefor); Fischbach et al. (Intracel), WO 90/01543 (method for the genetic expression of heterologous proteins by cells transfected); Robinson et al., seminars in IMMUNOLOGY, vol. 9, pp.271-283 (1997) (DNA vaccines); Szoka et al., U.S. Patent No. 4,394,448 (method of inserting DNA into living cells); and McCounick et al., U.S. Patent No. 5,677,178 (use of cytopathic viruses for therapy and prophylaxis of neoplasia).

The expression product generated by vectors or recombinants in this invention can also be isolated from infected or transfected cells and used to prepare compositions for administration to patients.

More generally, compositions for use in the invention can be prepared in accordance with standard techniques well known to those skilled in the pharmaceutical or medical arts. Such compositions can be administered in dosages and by techniques well known to those skilled in the medical arts taking into consideration such factors as the age, sex, weight, and condition of the particular patient, and the route of administration. The compositions can be administered alone, or can be co-administered or sequentially administered with other prophylactic or therapeutic compositions.

Examples of compositions of the invention include liquid preparations for orifice, e.g., oral, nasal, anal, genital (e.g., vaginal), vascular and/or SMC, etc., administration such as suspensions, syrups or elixirs; and, preparations for parenteral, subcutaneous, intradermal, intramuscular, intravenous, intraarterial (e.g., at site of lesion or plaque), intralymphatic, or intraperitoneal administration (e.g., injectable administration) such as sterile suspensions or emulsions. In such compositions the active agent be in admixture with a suitable carrier, diluent, or excipient such as sterile water, physiological saline, glucose or the like.

The compositions of the invention may be packaged in a single dosage form for immunization by parenteral (i.e., intramuscular, intradermal or subcutaneous) administration or orifice administration, e.g., perlingual (i.e., oral), intragastric, mucosal including intraoral, intraanal, intravaginal intravenous, intralymphatic, intraarterial (e.g., at site of lesion or plaque), intraperitoneal, and the like administration. Accordingly, compositions in forms for such administration routes are envisioned by the invention. And again, the effective dosage and route of administration are determined by known factors, such as age, sex, weight, condition and nature of patient, as well as LD₅₀ and other screening procedures which are known and do not require undue experimentation.

Dosages of each active agent can range from a few to a few hundred micrograms, e.g., 5 to 500 µg. Other suitable carriers or diluents can be waler or a buffered saline, with or without a preservative. The expression product or isolated product may be lyophilized for resuspension at the time of administration or can be in solution.

For treatment of restenosis, the compositions comprising the soluble VEGF receptor angiopoietin-1, alone or with other treatment, may be administered as desired by the skilled medical practitioner, from this disclosure and knowledge in the art, e.g., at the first signs or symptoms of restenosis, or as soon thereafter as desired by the skilled medical practitioner, without any undue experimentation required; and, the administration of the compositions, alone or with other treatment, may be continued as a regimen, e.g., monthly, bi-monthly, biannually, annually, or in some other regimen, by the skilled medical practitioner for such time as is necessary to prevent further clogging of blood vessels or further symptoms or signs of restenosis, without any undue experimentation required.

For treatment of atherosclerosis, the compositions, alone or with other treatment, may be administered at the first signs or symptoms of atherosclerosis, or as soon thereafter as desired by the skilled medical practitioner, without any undue experimentation required; and, the administration of the compositions, alone or with other treatment, may be continued as a regimen, e.g., monthly, bi-monthly, biannually, annually, or in some other regimen, by the skilled medical practitioner for such time as is necessary to prevent further clogging of blood vessels or further symptoms or signs of atherosclerosis, without any undue experimentation required.

The compositions of the invention can be administered before, during or immediately after the angioplasty to induce maximal responses at the time of angioplasty, since the restenotic process happens quickly.

A better understanding of the present invention and of its many advantages will be had from the following examples, given by way of illustration.

### EXAMPLES

### EXAMPLE1 - Atherogenesis

Microvascular angiogenesis (expansion of the vasovasorum) occurs during atherogenesis in the apoE knockout mouse, and the coordinated sequential expression of VEGF and ang-1, with activation of their signaling cascades, are consistent components of the microvascular angiogenic process (see Fig 1).

The vessels of apoE knockout mice are compared to those of the parental nonatherosclerostic strain.

Endpoint Measurements: To determine whether the VEGF and ang-1 signaling cascades are activated during atherogenesis, vessels are obtained from the parental noa-atheroscJeiotic mice and compared to vessels obtained at various timepoints from apoE knockout mice and analysed for one or more or any or all of:
- ang-1 protein (by immunohistochemistry and/or by Western analysis);
- tyrosine kinase phosphorylation of TIE 2 (to assess the state of activation of the receptor);
- VEGF protein (by immunohistochemistry and/or by Western analysis);
- tyrosine kinase phosphorylation of one or more of the VEGF receptors (to assess the state of activation of the receptor);
- atherosclerotic mass (measured by usual computerized image analysis tethniques);
- the magnitude of vasovasorum development measured by microscopic CT; and
- the magnitude of vasovasorum development measured by immunohistochemistry staining for endothelial cells.

These tests confirm Fig. 1.

Microvascular angiogenesis (expansion of the vasovasorum) is a critical determinant of the degree of atherosclerosis, and the coordinated sequential expression of VEGF and ang-1, and for their receptors, with activation of their signaling cascades, are necessary components of the angiogenic process occurring during atherogenesis. Moreover, a) upregulation of VEGF is necessary to destabilize a mature vessel to enable it to begin the angiogenic process, and b) increased activity of ang-1 without VEGF causes vessel maturation and stabilization, and therefore inhibits ongoing angiogenesis (Fig 1). Therefore, administration to apoE knockout mice of 1) an agent inhibiting VEGF (e.g., the soluble VEGF receptor) and 2) an agent inducing vessel maturation (e.g., ang-1) will reduce microangiogenesis and, thereby, atherosclerosis and/or restenosis (see Fig 2).

ApoE knockout mice are treated by intraperitoneal administration of the soluble VEGF receptor and with ang-1. These agents are administered as frequently as possible, with the maximal amount determined by the LD50 and by the availability of protein.

Endpoint Measurements: To determine whether the proposed strategy has had biologic effects, vessels are obtained at various timepoints from apoE knockout mice either treated or not treated as indicated and analysed for one or more or any or all of:
- ang-1 protein (by immunohistochemistry and/or by Western analysis);
- tyrosine kinase phosphorylation of TIE 2 (to assess the state of activation of the receptor);
- VEGF protein (by immunohistochemistry and/or by Western analysis);
- tyrosine kinase phosphorylation of one or more of the VEGF receptors (to assess the state of activation of the receptor);
- atherosclerotic mass (measured by usual computerized image analysis techniques);
- the magnitude of vasovasorum development measured by microscopic CT; and
- the magnitude of vasovasorum development measured by immunohistochemistry staining for endothelial cells.

The results confirm the foregoing.

### EXAMPLE 2 - Restenosis

Microangiogenesis (expansion of the vasovasorum) occurs during neointimal development following angioplasty (with or without stems), and the coordinated sequential expression of VEGF and ang-1, with activation of their signaling cascades, are consistent components of the microvascular angiogenic process.

The coronary vessels of pigs are injured by balloon angioplasty with or without stent implantation. To determine whether the VEGF and ang-1 signaling cascades are activated following vessel injury, vessels are obtained from each of 2 pigs sacrificed 2 h, 6 h, 24 h, 14 days and 28 days after injury and analysed.

Endpoint Measurements are one or more or any or all of:
- ang-1 protein (by immunohistochemistry and/or by Western blot);
- tyrosine kinase phosphorylation of TIE 2 (to assess the state of activation of the receptor);
- VEGF protein (by immunohistochemistry and/or by Western analysis);
- tyrosine kinase phosphorylation of one or more of the VEGF receptors (to assess the state of activation of the receptor);
- neointimal mass (measured by usual computerized image analysis techniques);
- the magnitude of vasovasorum development measured by microscopic CT; and
- the magnitude of vasovasorum development measured by immunohistochemistry staining for endothelial cells.

The results confirm the foregoing.

Microvascular angiogenesis (expansion of the vasovasorum) is a critical determinant of neointimal expansion and therefore of restenosis mass, and the coordinated sequential expression of VEGF and ang-1, and/or their receptors, with activation of their signaling cascades, are necessary components of the restenotic process occurring following vessel injury. Moreover, a) upregulation of VEGF is necessary to destabilize a mature vessel to enable it to begin the angiogenic process, and b) increased activity of ang-1 without VEGF causes vessel maturation and stabilization, and therefore inhibits ongoing angiogenesis (see Fig 1). Therefore, administration to the injured vessel wall of the soluble VEGF receptor and ang-1 will reduce microangiogenesis and, thereby, will reduce neointimal development (see Fig 2).

### EXAMPLE 3- Formulations and Use

The soluble VEGF receptor ang-1 are admixed with carrier, diluent etc., as herein described in amounts as herein described to obtain formulations. Patients are administered the formulations as herein described for the treatment of atherosclerosis and/or restenosis, including in a manner analogous to gene therapy directed against SMC proliferation, as described in literature cited herein or in documents cited in literature cited herein.

### REFERENCES

Barger et al., "Hypothesis: vasavasorum and neovascularization of human coronary arteries. A possible role in the pathophysiology of atherosclerosis." N Eng J Med 310(3):175-7 (Jan. 1984).
Koblizek et al. Curr Biol 8(9):529-32 (April 1998).
Witzenbichler et al. J Biol Chem 273(29):18514-21 (July 1998).
Asahara et al. Cir Res 83(3):233-40 (August 1998).
Suri et al., Science 282(5388):468-71 (October 1998).
Cheung et al. Genomics 48(3):389-91 (March 1998).
Maisonpierre et al. Science 277(5322):55-60 (July 1997).
Suri et al. Cell 87(7):1171-80 (December 1996).
Takahashi et al. Jpn J Cancer Res 89(4):445-51 (April 1998).
Raymond Presse Med 27(24):1221-4 (July 1998).
Folkman, Nature 390:404-7 (1997).
Parenti et al. J Biol Chem 272(7):4220-6 (February 1998).
Chua et al. Biochim Biophys Acta 1401(2):187-94 (February 1998).
Satake et al. Biol Cell 90(2):161-8 (March 1998).
Giraudo et al. J Biol Chem 273(34):22128-35 (August 1998).
Witzenbichler et al. Am J Pathol 153(2):381-94 (August 1998).
Metais et al. Am J. Physiol 275(4 Pt 2):H1411-8 (October 1998).
Agani et al. Mol Pharmacol 54(5):749-54 (November 1998).
Pepper et al. J Cell Physiol 177(3):439-52 (December 1998).
Chua et al. Free Rad Biol Med 25(8):891-7 (November 1998).
Parast et al. Biochemistry 37(47):16788-801 (November 1998).
Breier et al. Thromb Haemost 78(1):678-83 (July 1997).
Igarashi et al. Int J Mol Med 2(2):211-215 (August 1998).
WO 98/33510.
Kwon et al., Journal of Clinical Investigation, 101(8): 1551-56 (April 1998).
Kwon et al., "Adventitial Vasa Vasorum in Balloon-injured Coronary Arteries: Visualization and Quantitation by a Microscopic Three-dimensional Computed Tomography Technique" (1998).
Inoue et al., Circulation 98:2108-2116 (November 1998).
Marx, Science, Vol. 265 page 320 (July 15, 1994).
Epstein et al., JACC Vol. 23, No. 6,1994:1278-88.
Flugelman et al., "Smooth muscle cell abundance and fibroblast growth factors in coronary lesions of patients with nonfatal unstable angina. A clue to the mechanism of transformation from the stable to the unstable clinical stale." Circulation 88(6):2493-500 (Dec. 1993).
Chang et al., Science 267:518-22 (January 27, 1995).
French Patent Application 2723697.
Koblizek et al., current Biology 1998, 8: 529-532.
Korpelainen and Alitalo, current opinion in cell Biology 1998, 10:163-69 WO 98/33510 from Pasteur Mérieux serums et vaccins.

## Claims

1. A composition for therapy for restenosis and/or atherosclerosis comprising the soluble VEGF receptor and anglopoletin-1.

2. Use of the soluble VEGF receptor and angiopoietin-1 for the preparation of a composition for treating restenosis and/or atherosclerosis.

3. A kit for treating atherosderosis or restenosis comprising the soluble VEGF receptor and angiopoletln-1.

4. The kit of claim 3 wherein the soluble VEGF receptor and the anglopoletin-1 are in separate containers.

5. The kit of claim 4 wherein the separate containers are In a package together.

## Patentansprüche

1. Zusammensetzung zur Restenose- und/oder Atherosklerosetherapie, welche Zusammensetzung den löslichen VEGF-Rezeptor und Angiopoietin-1 umfasst.

2. Anwendung des löslichen VEGF-Rezeptors und Angiopoietin-1 zur Herstellung einer Zusammensetzung zur Behandlung von Restenose und/oder Atherosklerose.

3. Kit zur Behandlung von Atherosklerose oder Restenose, welcher Kit den löslichen VEGF-Rezeptor und Angiopoietin-1 umfasst.

4. Kit nach Anspruch 3, worin der lösliche VEGF-Rezeptor und das Angiopoietin-1 in getrennten Behältern vorliegen.

5. Kit nach Anspruch 4, worin die getrennten Behälter zusammen in einer Packung vorliegen.

## Revendications

1. Composition pour la thérapie de la resténose et/ou de l'athérosclérose comprenant un récepteur au VEGF soluble et l'angiopoïétine-1.

2. Utilisation du récepteur au VEGF soluble et de l'angiopoïétine-1 pour la préparation d'une composition pour le traitement de la resténose et/ou de l'athérosclérose.

3. Kit de traitement de l'athérosclérose ou de la resténose comprenant le récepteur au VEGF soluble et l'angiopdiétine-1.

4. Kit selon la revendication 3, dans lequel le récepteur au VEGF soluble et l'angiopoïétine-1 sont dans des containers séparés.

5. Kit selon la revendication 4, dans lequel les containers séparés sont dans le même conditionnement.
